# EUROPEAN PATENT SPECIFICATION

(11) **EP 3 840 637 B1**
(45) Date of publication and mention of the grant of the patent: **06.08.2025**
(21) Application number: 19852768.1
(22) Date of filing: 20.08.2019
(51) Int. Cl.: A61B 5/00, A61B 5/145, A61B 5/1459, A61B 5/1473, A61B 5/1455, A61K 31/192, A61K 31/573, A61K 31/60, A61K 47/54, A61K 47/69, G01N 33/52

(54) **MEDIATED DRUG RELEASE FOR REDUCING IN VIVO ANALYTE INDICATOR DEGRADATION**
VERMITTELTE WIRKSTOFFABGABE ZUR VERRINGERUNG DES IN-VIVO-ANALYTINDIKATORABBAUS
LIBÉRATION DE MÉDICAMENT INDUITE POUR RÉDUIRE LA DÉGRADATION D'UN INDICATEUR D'ANALYTE IN VIVO

(30) Priority: 20.08.2018 US 201862719927 P
(43) Date of publication of application: 30.06.2021
(73) Proprietor: Senseonics, Incorporated, Germantown, MD 20876 (US)
(72) Inventor: VELVADAPU, Venkata, Germantown, MD 20876 (US); HUFFSTETLER, Philip, Germantown, MD 20876 (US)
(74) Representative: Weickmann & Weickmann PartmbB
(86) International application number: PCT/US2019/047155
(87) International publication number: WO 2020/041240

(56) References cited:
- EP-A1- 2 534 470
- EP-A1- 2 744 909
- EP-B1- 2 534 470
- EP-B1- 2 744 909
- US-A1- 2006 281 185
- US-A1- 2007 014 726
- US-A1- 2008 146 803
- US-A1- 2015 057 509
- US-A1- 2015 057 509
- US-A1- 2017 311 897
- US-A1- 2018 220 940
- US-A1- 2019 159 708
- US-B2- 9 427 181
- DANIEL BUENGER ET AL: "Hydrogels in sensing applications", PROGRESS IN POLYMER SCIENCE, vol. 37, no. 12, 1 December 2012 (2012-12-01), GB, pages 1678 - 1719, XP055368137, ISSN: 0079-6700, DOI: 10.1016/j.progpolymsci.2012.09.001
- CHENG CHEN ET AL: "Current and Emerging Technology for Continuous Glucose Monitoring", SENSORS, vol. 17, no. 12, 19 January 2017 (2017-01-19), pages 182, XP055480591, DOI: 10.3390/s17010182

## Description

### CROSS-REFERENCE TO RELATED APPLICATION

The present application claims the benefit of priority to U.S. Provisional Application Serial No. 62/719,927, filed on August 20, 2018.

### BACKGROUND

### Field of Invention

The present invention relates generally to continuous reduction of *in vivo* degradation of analyte sensor moieties when measuring an analyte in a medium of a living animal using a system including a sensor implanted (partially or fully) or inserted into the living animal. Specifically, the present invention relates to a sensor that utilizes one or more boronic acid-drug conjugates to reduce degradation.

### Discussion of the Background

A sensor may be implanted (partially or fully) within a living animal *(e.g.,* a human) and used to measure an analyte *(e.g.,* glucose, oxygen, cardiac markers, low-density lipoprotein (LDL), high-density lipoprotein (HDL), or triglycerides) in a medium *(e.g.,* interstitial fluid (ISF), blood, or intraperitoneal fluid) within the living animal. The sensor may include a light source *(e.g.,* a light-emitting diode (LED) or other light emitting element), indicator molecules, and a photodetector *(e.g.,* a photodiode, phototransistor, photoresistor or other photosensitive element). Examples of implantable sensors employing indicator molecules to measure an analyte are described in U.S. Pat. Nos. 5,517,313 and 5,512,246; following documents also refer to implantable analyte sensors: US 2015/057509 A1, DANIEL BUENGER ET AL: "Hydrogels in sensing applications", PROGRESS IN POLYMER SCIENCE, vol. 37, no. 12, 1 December 2012 (2012-12-01), pages 1678-1719, GB ISSN: 0079-6700, DOI: 10.1016/ j.progpolymsci.2012.09.001, EP 2 744 909 A1, US 2007/014726 A1, EP 2 534 470 A1.

A sensor may include an analyte indicator, which may be in the form of indicator molecules embedded in a graft *(i.e.,* layer or matrix). For example, in an implantable fluorescence-based glucose sensor, fluorescent indicator molecules may reversibly bind glucose and, when irradiated with excitation light *(e.g.,* light having a wavelength of approximately 378 nm), emit an amount of light *(e.g.,* light in the range of 400 to 500 nm) that depends on whether glucose is bound to the indicator molecule.

If a sensor is implanted in the body of a living animal, the animal's immune system may begin to attack the sensor. For instance, if a sensor is implanted in a human, white blood cells may attack the sensor as a foreign body, and, in the initial immune system onslaught, neutrophils may be the primary white blood cells attacking the sensor. The defense mechanism of neutrophils includes the release of highly caustic substances known as reactive oxygen species. The reactive oxygen species include, for example, hydrogen peroxide. As used herein, the terms "degradative species" and "biological oxidizers" generally refer to reactive physiological molecules and radicals that degrade the indicator molecules.

Hydrogen peroxide and other degradative species such as reactive oxygen and nitrogen species may degrade the indicator molecules of an analyte indicator. For instance, in indicator molecules having a boronate group, hydrogen peroxide may degrade the indicator molecules by oxidizing the boronate group, thus disabling the ability of the indicator molecule to bind glucose. The longevity of certain implantable sensors is achieved in part or in whole using anti-inflammatory drugs such as dexamethasone. In conventional sensors that use anti-inflammatory drugs, there is a constant rate of drug elution for patients with both low- and elevated-levels of oxidative stress. As such, the drug is not effectively utilized, leading to a short than desired sensor lifetime.

There is presently a need in the art for improvements in reducing analyte indicator degradation. There is also a need in the art for continuous analyte sensors having increased longevity.

### SUMMARY

The present invention overcomes the disadvantages of prior systems by providing, among other advantages, reduced analyte indicator degradation. The invention is defined in the appended claims.

One aspect of the present invention provides a sensor for measurement of an analyte in a medium within the living animal as defined in independent claim 1.

In some embodiments, the at least one boronic acid-drug conjugate may include a boronic acid-dexamethasone conjugate.

In some embodiments, the sensor may be implantable within a living animal. In some embodiments, the sensor may further include at least one drug eluting polymer matrix covering at least a portion of the sensor housing, and the boronic acid-drug conjugate may be dispersed within the drug eluting polymer matrix. In some embodiments, the drug eluting polymer matrix may have a preformed shape. In some embodiments, the preformed shape may be a ring, a sleeve, a conformal shell, a cylinder, or a monolith. In some embodiments, the drug eluting polymer matrix may be adjacent to the analyte indicator.

In some embodiments, the boronic acid-drug conjugate may be a co-monomer with the analyte indicator. In some embodiments, the boronic acid-drug conjugate may be a co-monomer with the analyte indicator in a hydrogel. In some embodiments, the boronic acid-drug conjugate may be configured to reduce oxidation of the analyte indicator. In some embodiments, the boronic acid-drug conjugate may be configured to interact or react with a degradative species without compromising signal integrity or performance of the sensor device, and the degradative species may be hydrogen peroxide, a reactive oxygen species, a reactive nitrogen species, or a free radical. In some embodiments, the drug of the boronic acid-drug conjugate may be conjugated to the boronic acid moiety via covalent bonds that are stable in the absence of a degradative species. In some embodiments, the drug of the boronic acid-drug conjugate may be conjugated to the boronic acid moiety via covalent bonds that break in the presence of a degradative species and release the drug.

In some embodiments, the drug may be an anti-inflammatory drug. In some embodiments, the anti-inflammatory drug may be a non-steroidal anti-inflammatory drug. In some embodiments, the non-steroidal anti-inflammatory drug may be acetylsalicylic acid. In some embodiments, the non-steroidal anti-inflammatory drug may be isobutylphenylpropanoic acid. In some embodiments, the drug may be a glucocorticoid. In some embodiments, the drug may be dexamethasone, triamcinolone, betamethasone, methylprednisolone, beclometasone, fludrocortisone, a derivative thereof, an analog thereof, or a combination of two or more thereof.

In some embodiments, the analyte indicator may be a graft including indicator molecules. In some embodiments, the sensor may further include a layer of a catalyst capable of converting hydrogen peroxide into water and oxygen on at least a portion of the analyte indicator. In some embodiments, the sensor may further include a membrane covering at least a portion of the analyte indicator. In some embodiments, the membrane may be a porous, opaque diffusion membrane.

In some embodiments, the boronic acid-drug conjugate may be formed by conjugating a boronic acid compound of Formula I to the drug, Formula I may be: 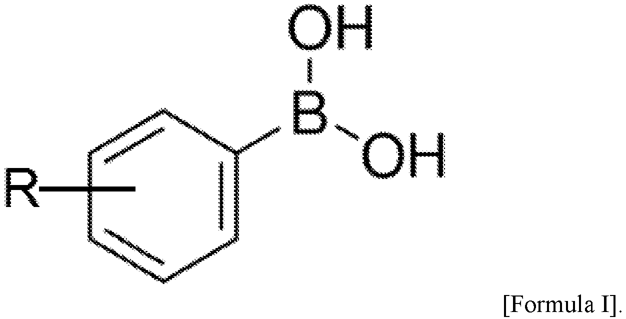 One or more R may be independently selected from hydrogen, hydroxyl, an alkyl group, an alkenyl group, an alkynyl group, a halo group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the boronic acid-drug conjugate may be: 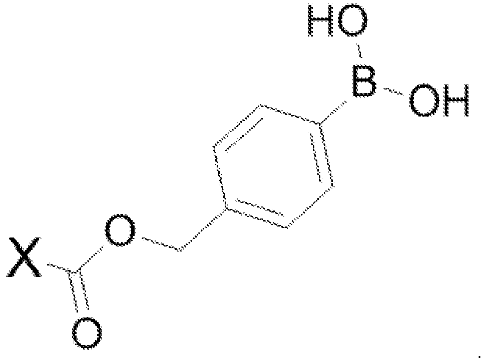 X may be the drug or a linking moiety connecting the boronic acid moiety to the drug, the linking moiety may be a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the boronic acid-drug conjugate may be the drug conjugated with one or more of the following compounds either directly or via a linking moiety: 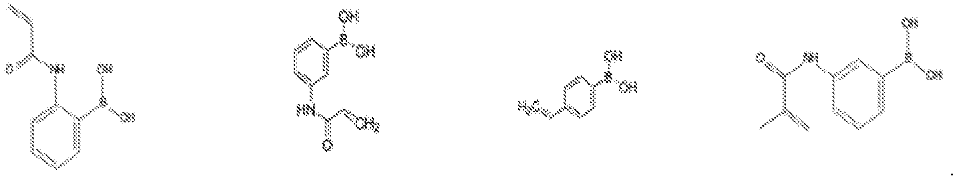 In a conjugate having the linking moiety, the linking moiety may be a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the boronic acid-drug conjugate may include the drug conjugated to [4-(2-carboxymethyl)phenyl]boronic acid.

Another aspect of the present invention provides a method of fabricating a sensor for measurement of an analyte in a medium within a living animal as defined in independent claim 15.

In some embodiments, the one or more boronic acid-drug conjugates may be co-monomers with the analyte indicator. In some embodiments, the one or more boronic acid-drug conjugates may be co-monomers with the analyte indicator in a hydrogel. In some embodiments, the drug may be an anti-inflammatory drug. In some embodiments, the anti-inflammatory drug may be a non-steroidal anti-inflammatory drug. In some embodiments, the non-steroidal anti-inflammatory drug may be acetylsalicylic acid. In some embodiments, the non-steroidal anti-inflammatory drug may be isobutylphenylpropanoic acid. In some embodiments, the drug may be a glucocorticoid. In some embodiments, the drug may be dexamethasone, triamcinolone, betamethasone, methylprednisolone, beclometasone, fludrocortisone, a derivative thereof, an analog thereof, or a combination of two or more thereof.

In some embodiments, the analyte indicator may be a graft including indicator molecules. In some embodiments, the method may further include applying a layer of a catalyst capable of converting hydrogen peroxide into water and oxygen on at least a portion of the analyte indicator. In some embodiments, the method may further include covering at least a portion of the analyte indicator with a membrane. In some embodiments, the membrane may be a porous, opaque diffusion membrane.

In some embodiments, the boronic acid-drug conjugate may be formed by conjugating a boronic acid compound of Formula I to the drug, Formula I may be: 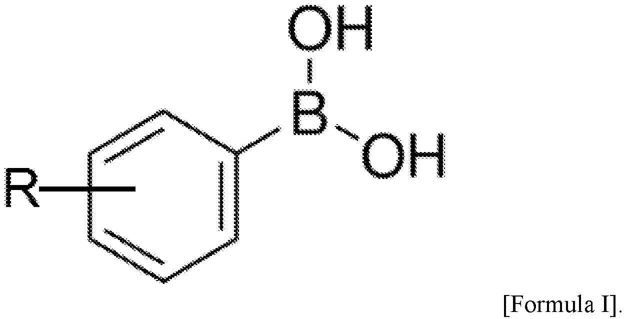 One or more R substituent may be independently selected from hydrogen, hydroxyl, an alkyl group, an alkenyl group, an alkynyl group, a halo group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the boronic acid-drug conjugate may be: 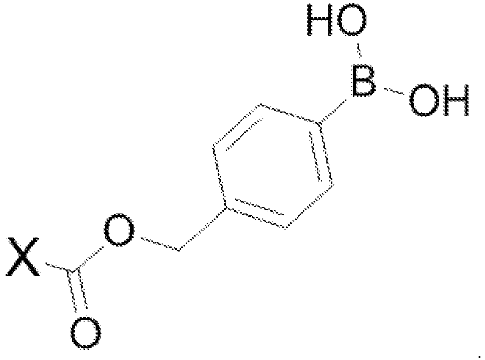 The X may be the drug or a linking moiety connecting the boronic acid moiety to the drug, the linking moiety may be a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the drug of the boronic acid-drug conjugate may be conjugated with one or more of the following compounds either directly or via a linking moiety: 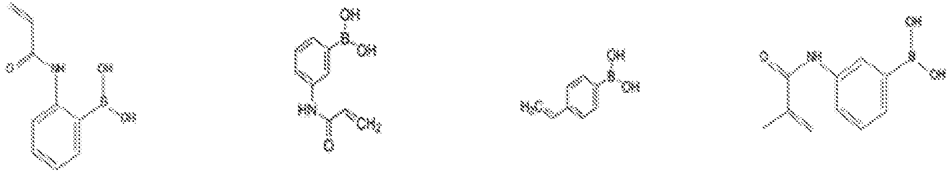 In a conjugate having the linking moiety, the linking moiety may be a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some embodiments, the drug of the boronic acid-drug conjugate may be conjugated to [4-(2-carboxymethyl)phenyl]boronic acid.

In some embodiments, the drug in the boronic acid-drug conjugate may be dexamethasone.

Yet another aspect, not being part of the present invention, may provide a method for detecting the presence or concentration of an analyte in an *in vivo* sample. The method may include exposing the *in vivo* sample to a device having a detectable quality that changes when the device is exposed to an analyte of interest. The device may include a boronic acid-drug conjugate that reacts with a degradative species or biological oxidizers to release drug from the boronic acid-drug conjugate, thereby preventing or reducing degradation or interference of the device from degradative species or biological oxidizers. The device may be the any of the sensors described above. The method may include measuring a change in the detectable quality to thereby detect the presence or concentration of the analyte of interest in the *in vivo* sample.

Further variations encompassed within the systems and methods are described in the detailed description of the invention below.

### BRIEF DESCRIPTION OF THE DRAWINGS

The accompanying drawings, which are incorporated herein and form part of the specification, illustrate various, non-limiting embodiments of the present invention. In the drawings, like reference numbers indicate identical or functionally similar elements.
FIG. 1 is a schematic view illustrating a sensor system embodying aspects of the present invention.
FIG. 2 illustrates a perspective view of a sensor embodying aspects of the present invention.
FIG. 3 illustrates an exploded view of a sensor embodying aspects of the present invention.
FIG. 4 shows a thin layer chromatography (TLC) plate showing release of dexamethasone from Compound A in the presence of hydrogen peroxide (rows B and C) and a control run of free dexamethasone (row A).

### DETAILED DESCRIPTION OF PREFERRED EMBODIMENTS

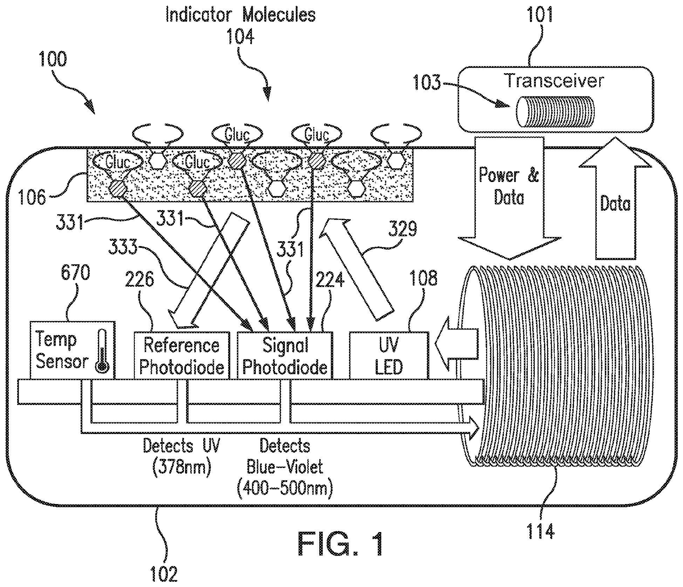

FIG. 1 is a schematic view of a sensor system embodying aspects of the present invention. In some non-limiting embodiment, as shown in FIG. 1, the system may include a sensor 100 and an external transceiver 101. In some embodiments, the sensor 100 may be an implantable sensor configured to be fully or partially implanted in a living animal *(e.g.,* a living human). The sensor 100 may be implanted, for example, in a living animal's arm, wrist, leg, abdomen, peritoneum, or other region of the living animal suitable for sensor implantation. For example, in some non-limiting embodiments, the sensor 100 may be implanted beneath the skin *(i.e.,* in the subcutaneous or peritoneal tissues). However, this is not required, and, in some alternative embodiments, the sensor 100 may be a transcutaneous sensor.

In some embodiments, a transceiver 101 may be an electronic device that communicates with the sensor 100 to power the sensor 100, provide commands and/or data to the sensor 100, and/or receive data from the sensor 100. In some embodiments, the received data may include one or more sensor measurements. In some embodiments, the sensor measurements may include, for example and without limitation, one or more light measurements from one or more photodetectors of the sensor 100 and/or one or more temperature measurements from one or more temperature sensors of the sensor 100. In some embodiments, the transceiver 101 may calculate analyte (e.g., glucose) concentrations from the measurement information received from the sensor 100.

In some non-limiting embodiments, the transceiver 101 may be a handheld device or an on-body/wearable device. For example, in some embodiments where the transceiver 101 is an on-body/wearable device, the transceiver 101 may be held in place by a band *(e.g.,* an armband or wristband) and/or adhesive, and the transceiver 101 may convey (e.g., periodically, such as every two minutes, and/or upon user initiation) measurement commands *(i.e.,* requests for measurement information) to the sensor 100. In some embodiments where the transceiver 101 is a handheld device, positioning *(i.e.,* hovering or swiping/waving/passing) the transceiver 101 within range over the sensor implant site *(i.e.,* within proximity of the sensor 100) may cause the transceiver 101 to automatically convey a measurement command to the sensor 100 and receive a data from the sensor 100.

In some embodiments, as shown in FIG. 1, the transceiver 101 may include an inductive element 103, such as, for example, a coil. In some embodiments, the transceiver 101 may generate an electromagnetic wave or electrodynamic field *(e.g.,* by using a coil) to induce a current in an inductive element 114 of the sensor 100. In some non-limiting embodiments, the sensor 100 may use the current induced in the inductive element 114 to power the sensor 100. However, this is not required, and, in some alternative embodiments, the sensor 100 may be powered by an internal power source (e.g., a battery).

In some embodiments, the transceiver 101 may convey data (*e.g*., commands) to the sensor 100. For example, in some non-limiting embodiments, the transceiver 101 may convey data by modulating the electromagnetic wave generated by the inductive element 103 *(e.g.,* by modulating the current flowing through the inductive element 103 of the transceiver 101). In some embodiments, the sensor 100 may detect/extract the modulation in the electromagnetic wave generated by the transceiver 101. Moreover, the transceiver 101 may receive data *(e.g.,* one or more sensor measurements) from the sensor 100. For example, in some non-limiting embodiments, the transceiver 101 may receive data by detecting modulations in the electromagnetic wave generated by the sensor 100, *e.g.,* by detecting modulations in the current flowing through the inductive element 103 of the transceiver 101.

In some embodiments, as shown in FIG. 1, the sensor 100 may include a sensor housing 102 *(i.e.,* body, shell, capsule, or encasement), which may be rigid and biocompatible. In exemplary embodiments, sensor housing 102 may be formed from a suitable, optically transmissive polymer material, such as, for example, acrylic polymers (*e.g.*, polymethylmethacrylate (PMMA)).

In some embodiments, as shown in FIG. 1, the sensor 100 may include an analyte indicator 106. In some non-limiting embodiments, the analyte indicator 106 may be a polymer graft coated, diffused, adhered, or embedded on at least a portion of the exterior surface of the sensor housing 102. The analyte indicator 106 (e.g., polymer graft) may cover the entire surface of sensor housing 102 or only one or more portions of the surface of housing 102. As an alternative to coating the analyte indicator 106 on the outer surface of sensor housing 102, the analyte indicator 106 may be disposed on the outer surface of the sensor housing 102 in other ways, such as by deposition or adhesion. In some embodiments, the analyte indicator 106 may be a fluorescent glucose indicating polymer. In one non-limiting embodiment, the polymer is biocompatible and stable, grafted onto the surface of sensor housing 102, designed to allow for the direct measurement of glucose in interstitial fluid (ISF), blood, or intraperitoneal fluid after implantation of the sensor 100. In some embodiments, the analyte indicator 106 may be a hydrogel.

In some embodiments, the analyte indicator 106 *(e.g.,* polymer graft) of the sensor 100 may include indicator molecules 104. The indicator molecules 104 may be distributed throughout the entire analyte indicator 106 or only throughout one or more portions of the analyte indicator 106. The indicator molecules 104 may be fluorescent indicator molecules (*e.g.*, TFM having the chemical name 9-[N-[6-(4,4,5,5,-tetramethyl-1,3,2-dioxaborolano)-3-(trifluoromethyl)benzyl]-N-[3-(methacrylamido)propylamino]methyl]-10-[N-[6-(4,4,5,5,-tetramethyl-1,3,2-dioxaborolano)-3-(trifluoromethyl)benzyl]-N-[2-(carboxyethyl)amino]methyl]anthracene sodium salt) or light absorbing, non-fluorescent indicator molecules. In some embodiments, the indicator molecules 104 may reversibly bind an analyte (*e.g*., glucose, oxygen, cardiac markers, low-density lipoprotein (LDL), high-density lipoprotein (HDL), or triglycerides). When an indicator molecule 104 has bound an analyte, the indicator molecule may become fluorescent, in which case the indicator molecule 104 is capable of absorbing (or being excited by) excitation light 329 and emitting light 331. In one non-limiting embodiment, the excitation light 329 may have a wavelength of approximately 378 nm, and the emission light 331 may have a wavelength in the range of 400 to 500 nm. When no analyte is bound, the indicator molecule 104 may be only weakly fluorescent.

In some embodiments, the sensor 100 may include a light source 108, which may be, for example, a light emitting diode (LED) or other light source that emits radiation, including radiation over a range of wavelengths that interact with the indicator molecules 104. In other words, the light source 108 may emit the excitation light 329 that is absorbed by the indicator molecules in the matrix layer/polymer 104. As noted above, in one non-limiting embodiment, the light source 108 may emit excitation light 329 at a wavelength of approximately 378 nm.

In some embodiments, the sensor 100 may also include one or more photodetectors (e.g., photodiodes, phototransistors, photoresistors or other photosensitive elements). For example, in the embodiment illustrated in FIG. 1, sensor 100 has a first photodetector 224 and a second photodetector 226. However, this is not required, and, in some alternative embodiments, the sensor 100 may only include the first photodetector 224. In the case of a fluorescence-based sensor, the one or more photodetectors may be sensitive to fluorescent light emitted by the indicator molecules 104 such that a signal is generated by a photodetector (e.g., photodetector 224) in response thereto that is indicative of the level of fluorescence of the indicator molecules and, thus, the amount of analyte of interest (*e.g*., glucose).

Some part of the excitation light 329 emitted by the light source 108 may be reflected from the analyte indicator 106 back into the sensor 100 as reflection light 333, and some part of the absorbed excitation light may be emitted as emitted (fluoresced) light 331. In one non-limiting embodiment, the emitted light 331 may have a different wavelength than the wavelength of the excitation light 329. The reflected light 333 and emitted (fluoresced) light 331 may be absorbed by the one or more photodetectors (e.g., first and second photodetectors 224 and 226) within the body of the sensor 100.

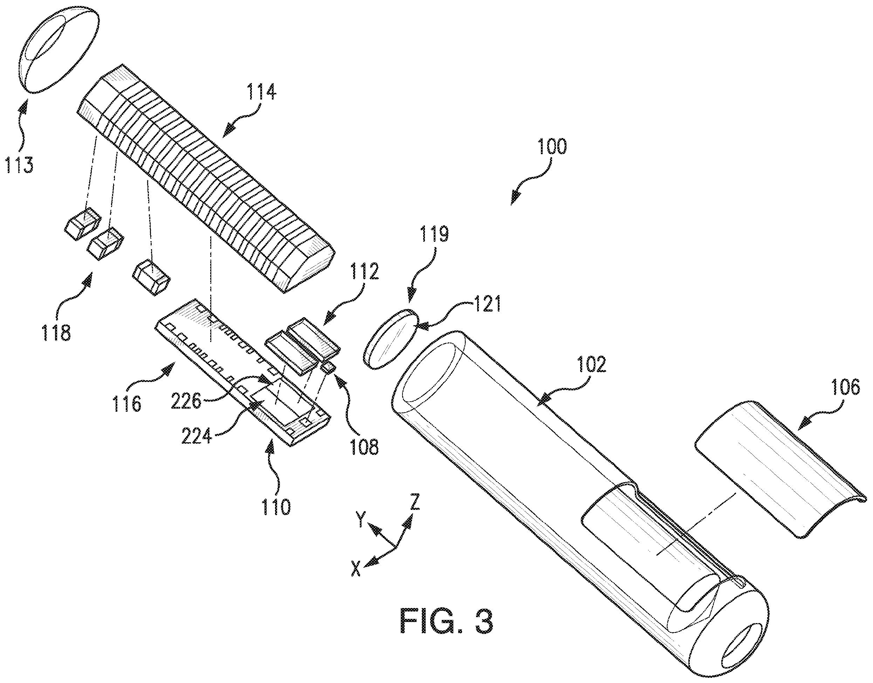

Each of the one or more photodetectors may be covered by a filter 112 (see FIG. 3) that allows only a certain subset of wavelengths of light to pass through. In some embodiments, the one or more filters 112 may be thin glass filters. In some embodiments, the one or more filters 112 may be thin film *(e.g.,* dichroic) filters deposited on the glass and may pass only a narrow band of wavelengths and otherwise reflect most of the received light. In some embodiments, the filters may be thin film (dichroic) filters deposited directly onto the photo detectors and may pass only a narrow band of wavelengths and otherwise reflect most of the light received thereby. The filters 112 may be identical (e.g., both filters 112 may allow signals to pass) or different *(e.g.,* one filter 112 may be a reference filter and another filter 112 may be a signal filter).

In one non-limiting embodiment, the second (reference) photodetector 226 may be covered by a reference photodiode filter that passes light at the same wavelength as is emitted from the light source 108 *(e.g.,* 378 nm). The first (signal) photodetector 224 may detect the amount of fluoresced light 331 that is emitted from the molecules 104 in the analyte indicator 106. In one non-limiting embodiment, the peak emission of the indicator molecules 104 may occur around 435 nm, and the first photodetector 224 may be covered by a signal filter that passes light in the range of about 400 nm to 500 nm. In some embodiments, higher glucose levels/concentrations correspond to a greater amount of fluorescence of the molecules 104 in the analyte indicator 106, and, therefore, a greater number of photons striking the first photodetector 224.

In some embodiments, as shown in FIG. 1, the sensor 100 may include a substrate 116. In some embodiments, the substrate 116 may be a circuit board *(e.g.,* a printed circuit board (PCB) or flexible PCB) on which circuit components (*e.g*., analog and/or digital circuit components) may be mounted or otherwise attached. However, in some alternative embodiments, the substrate 116 may be a semiconductor substrate having circuitry fabricated therein. The circuitry may include analog and/or digital circuitry. Also, in some semiconductor substrate embodiments, in addition to the circuitry fabricated in the semiconductor substrate, circuitry may be mounted or otherwise attached to the semiconductor substrate 116. In other words, in some semiconductor substrate embodiments, a portion or all of the circuitry, which may include discrete circuit elements, an integrated circuit *(e.g.,* an application specific integrated circuit (ASIC)) and/or other electronic components, may be fabricated in the semiconductor substrate 116 with the remainder of the circuitry is secured to the semiconductor substrate 116, which may provide communication paths between the various secured components.

In some embodiments, the one or more of the sensor housing 102, analyte indicator 106, indicator molecules 104, light source 108, photodetectors 224, 226, temperature transducer 670, substrate 116, and inductive element 114 of sensor 100 may include some or all of the features described in one or more of U.S. Application Serial No. 13/761,839, filed on February 7, 2013, U.S. Application Serial No. 13/937,871, filed on July 9, 2013, and U.S. Application Serial No. 13/650,016, filed on October 11, 2012. Similarly, the structure and/or function of the sensor 100 and/or transceiver 101 may be as described in one or more of U.S. Application Serial Nos. 13/761,839, 13/937,871, and 13/650,016.

In some embodiments, the sensor 100 may include a transceiver interface device, and the transceiver 101 may include a sensor interface device. In some embodiments where the sensor 100 and transceiver 101 include an antenna or antennas (*e.g*., inductive elements 103 and 114), the transceiver interface device may include the inductive element 114 of the sensor 100, and the sensor interface device may include the inductive element 103 of the transceiver 101. In some of the transcutaneous embodiments where there exists a wired connection between the sensor 100 and the transceiver 101, the transceiver interface device and sensor interface device may include the wired connection.

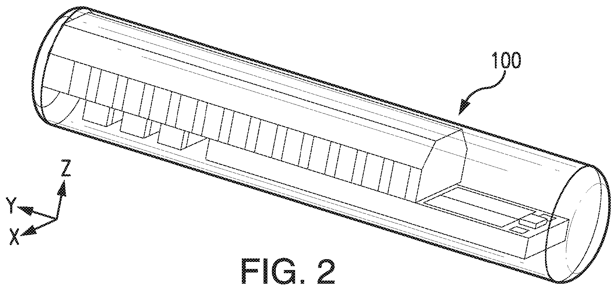

FIGS. 2 and 3 illustrate a non-limiting embodiment of a sensor 100 embodying aspects of the present invention that may be used in the sensor system illustrated in FIG. 1. FIGS. 2 and 3 illustrate perspective and exploded views, respectively, of the non-limiting embodiment of the sensor 100.

In some embodiments, as illustrated in FIG. 3, the sensor housing 102 may include an end cap 113. In some embodiments, the sensor 100 may include one or more capacitors 118. The one or more capacitors 118 may be, for example, one or more tuning capacitors and/or one or more regulation capacitors. The one or more capacitors 118 may be too large for fabrication in the semiconductor substrate 116 to be practical. Further, the one or more capacitors 118 may be in addition to one or more capacitors fabricated in the semiconductor substrate 116.

In some embodiments, as illustrated in FIG. 3, the sensor 100 may include a reflector *119 (i.e.,* mirror). Reflector 119 may be attached to the semiconductor substrate 116 at an end thereof. In a non-limiting embodiment, reflector 119 may be attached to the semiconductor substrate 116 so that a face portion 121 of reflector 119 is generally perpendicular to a top side of the semiconductor substrate 116 *(i.e.,* the side of semiconductor substrate 116 on or in which the light source 108 and one or more photodetectors 110 are mounted or fabricated) and faces the light source 108. The face 121 of the reflector 119 may reflect radiation emitted by light source 108. In other words, the reflector 119 may block radiation emitted by light source 108 from exiting the axial end of the sensor 100.

According to one aspect of the invention, an application for which the sensor 100 was developed (although by no means the only application for which it is suitable) is measuring various biological analytes in the living body of an animal (including a human). For example, sensor 100 may be used to measure glucose, oxygen, toxins, pharmaceuticals or other drugs, hormones, and other metabolic analytes in, for example, the human body.

In some embodiments, the specific composition of the analyte indicator 106 and the indicator molecules 104 may vary depending on the particular analyte the sensor is to be used to detect and/or where the sensor is to be used to detect the analyte (e.g., in the in subcutaneous tissues, blood, or peritoneum). In some embodiments, the analyte indicator 106 facilitates exposure of the indicator molecules 104 to the analyte. In some embodiments, the indicator molecules 104 may exhibit a characteristic (e.g., emit an amount of fluorescence light) that is a function of the concentration of the specific analyte to which the indicator molecules 104 are exposed.

The implantation or insertion of a medical device, such as a bio-sensor, into a user/patient's body can cause the body to exhibit adverse physiological reactions that are detrimental to the functioning of the device. The reactions may range from infections due to implantation surgery to the immunological response of a foreign object implanted in the body. That is, the performance of the implantable bio-sensor can be hindered or permanently damaged *in vivo* via the immunological response to an infection or the device itself. In particular, the performance of the analyte indicator 106 may be deteriorated by the immunological response of the body into which the sensor 100 is implanted. For example, as explained above, white blood cells, including neutrophils, may attack an implanted sensor 100. The neutrophils release degradative species including, *inter alia,* hydrogen peroxide, which may degrade indicator molecules 104 *(e.g.,* by oxidizing a boronate group of an indicator molecule 104 and disabling the ability of the indicator molecule 104 to bind glucose and/or fluoresce). In some embodiments, degradative species may include one or more of hydrogen peroxide, a reactive oxygen species, a reactive nitrogen species, and a free radical.

In some embodiments, the sensor 100 may include one or more boronic acid-drug conjugates that interact or react with one or more degradative species without compromising signal integrity or performance of the sensor device. In some non-limiting embodiments, the one or more boronic acid-drug conjugates may be conjugates of phenylboronic acid compounds with drugs that interact with degradative species without compromising signal integrity or performance of the sensor. In some non-limiting embodiments, the parent drug used to form one or more of the boronic acid-drug conjugates may be dexamethasone, triamcinolone, betamethasone, methylprednisolone, beclometasone, fludrocortisone, derivatives thereof, and analogs thereof, a glucocorticoid, or an anti-inflammatory drug (e.g., a non-steroidal anti-inflammatory drug including but not limited to acetylsalicylic acid, isobutylphenylpropanoic acid).

In some embodiments, the parent drug may be modified with aryl boronic acid moiety which undergoes oxidation by consuming the ROS (acting as a sacrificial boronic acid). Oxidation may rearrange the phenol, which may release the parent drug, lead to the drug action, and lead to a longer sensor life. In some embodiments, a boronic acid-drug conjugate may provide a unique release profile of the parent drug, and the amount of the parent drug released may be proportional to the extent of oxidation. In some embodiments, the boronic acid-drug conjugate may be configured such that an oxidative burst causes release of the drug from the conjugate. Thus, unlike conventional sensors having a constant rate of drug elution, the boronic acid-drug conjugate may release the drug when it is needed and in proportion to the oxidative conditions surrounding the sensor 100. Accordingly, the boronic acid-drug conjugate may advantageously extend the lifetime of implantable sensors.

In some non-limiting embodiments, a sensor 100 for measurement of an analyte (e.g., glucose) in a medium (e.g., interstitial fluid) within a living animal (e.g., a human) may include a sensor housing 102 and an analyte indicator 106. In some embodiments, the analyte indicator may include one or more indicator molecules 104, which may be distributed throughout the analyte indicator 106. In some embodiments, the indicator molecules 104 may be configured to reversibly bind the analyte. In some embodiments, the analyte indicator 106 may cover at least a portion of the sensor housing 102. In some embodiments, the sensor 100 may include a light source 108 (e.g., within the sensor housing 102) configured to emit excitation light 329. In some embodiments, the indicator molecules 104 may configured to be irradiated by the excitation light 329 and emit light 331 indicative of the amount of the analyte in the medium within the living animal. In some embodiments, the sensor 100 may include a photodetector 224 (e.g., within the sensor housing 102) that is sensitive to light 331 emitted by the one or more indicator molecules 104 and configured to generate a signal indicative of the amount of the analyte in the medium within the living animal.

In some embodiments, the sensor 100 may include one or more boronic acid-drug conjugates. In some embodiments the one or more boronic acid-drug conjugates may be configured to interact with degradative species. In some embodiments, the one or more boronic acid-drug conjugates may protect indicator molecules 104 of the analyte indicator 106 by preventing or reducing degradation or interference caused by degradative species or biological oxidizers. In some embodiments, the one or more boronic acid-drug conjugates may protect the indicator molecules 104 without compromising signal integrity or performance of the sensor 100.

In some embodiments, the sensor 100 may include at least one drug eluting polymer matrix and/or a layer of catalyst that may be provided on, incorporated in, or dispersed within the analyte indicator or sensor housing as described in U.S. Pat. No. 9,931,068 (Huffstetler et al .). In some embodiments, one or more boronic acid-drug conjugates may be incorporated in the analyte indicator 106. In some embodiments, the sensor 100 may include a membrane covering at least a portion of the analyte indicator 106, and the one or more boronic acid-drug conjugates may be incorporated within the membrane. In some embodiments, the sensor 100 may include a drug-eluting layer covering at least a portion of the analyte indicator 106, wherein the drug-eluting layer includes one or more boronic acid-drug conjugates. In some embodiments, the drug of the boronic acid-drug conjugate may be one or more of dexamethasone, triamcinolone, betamethasone, methylprednisolone, beclometasone, fludrocortisone, derivatives thereof, and analogs thereof, a glucocorticoid, and an anti-inflammatory drug (e.g., a non-steroidal anti-inflammatory drug including but not limited to acetylsalicylic acid, isobutylphenylpropanoic acid).

In some embodiments, the at least one drug eluting layer may include a membrane, mesh, nylon, fabric, polymer material, sponge, or other pore-containing material.
In some embodiments, one or more boronic acid-drug conjugates may be incorporated into the analyte indicator 106 that may cover at least a portion of the sensor housing 102. In some embodiments, the boronic acid-drug conjugate is a co-monomer with the analyte indicator, for example in a hydrogel.

In some embodiments, one or more boronic acid-drug conjugates may additionally or alternatively be in a drug eluting polymer matrix, which may be as described in U.S. Pat. No. 9,931,068 (Huffstetler et al.). In some embodiments, the drug eluting polymer matrix may cover a portion of the sensor housing 102. In some non-limiting embodiments, the drug-eluting polymer matrix may be applied to the sensor housing 102 via dip coating. In some non-limiting embodiments, as an alternative to dip coating, the drug-eluting polymer matrix may be applied to the sensor housing 102 via spray coating. In some non-limiting embodiments, as an alternative to a dip or spray coated drug-eluting polymer matrix, the drug-eluting polymer matrix may have a pre-formed shape such as, for example, a ring or sleeve. Other pre-formed shapes are possible, such as, for example and without limitation, a shell (e.g., conformal shell), cylinder, or any suitable monolith (e.g. rectangular).

One or more types of boronic acid-drug conjugates may be dispersed within the drug eluting polymer matrix (e.g., an inert polymer matrix). In some embodiments, the one or more the boronic acid-drug conjugates may reduce or stop the migration of neutrophils from entering the insertion site and, thus, reduce or stop the production of hydrogen peroxide and fibrotic encapsulation. In some embodiments, the one or more boronic acid-drug conjugates may be provided in the analyte indicator 106 (e.g., polymer graft). In some embodiments, the one or more boronic acid-drug conjugates may interact and/or react with degradative species. In some embodiments, the one or more boronic acid-drug conjugates may neutralize the degradative species. In some embodiments, the one or more boronic acid-drug conjugates may bind to the degradative species. In some embodiments, the one or more boronic acid-drug conjugates may sequester the degradative species so as to inhibit, reduce, and/or prevent degradation of the analyte indicator by the degradative species. Accordingly, in some embodiments, the one or more boronic acid-drug conjugates reduce deterioration of the analyte indicator 106.

In some non-limiting embodiments, one or more of the boronic acid compounds used in forming the boronic acid-drug conjugates may be a compound of Formula I:

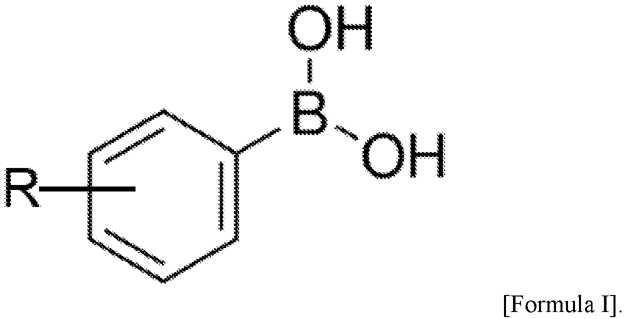

In some embodiments, one or more R groups attached to the phenyl ring may be independently selected from hydrogen, hydroxyl, an alkyl group, an alkenyl group, an alkynyl group, a halo group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂. In some embodiments, R₁ and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

In some non-limiting examples, the one or more boronic acid compounds may include the following compound: 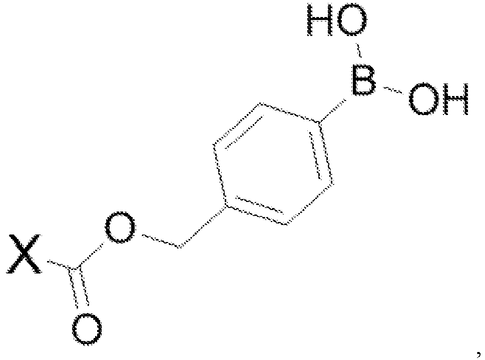 wherein X is the parent drug or a linking moiety connected to the parent drug. In some non-limiting embodiments, the linking moiety may be selected from a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂.

In some non-limiting examples, the parent drug may be conjugated with one or more of the following compounds either directly or via a linking moiety, e.g., as defined above:

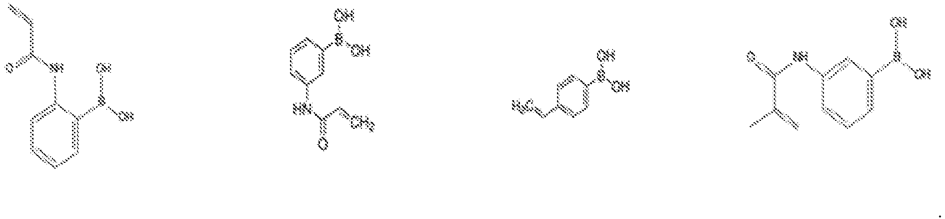

A sensor having one or more boronic acid-drug conjugates may have improved performance over a sensor that does not include a boronic acid-drug conjugate-containing analyte indicator. For instance, in some non-limiting embodiments, the boronic acid-drug conjugate may improve the longevity and functionality of the sensor 100.

### EXAMPLE 1

Illustrated below is a reaction scheme showing the parent drug ("Target") sequestered when it is conjugated with a boronic acid moiety. The presence of a reactive species in the environment of the sensor, e.g., hydrogen peroxide after an oxidative burst, causes a series of reactions resulting in consumption of the reactive species by the boronic acid moiety, followed by release of the parent drug leading to the drug action which would increase the lifetime of the sensor.

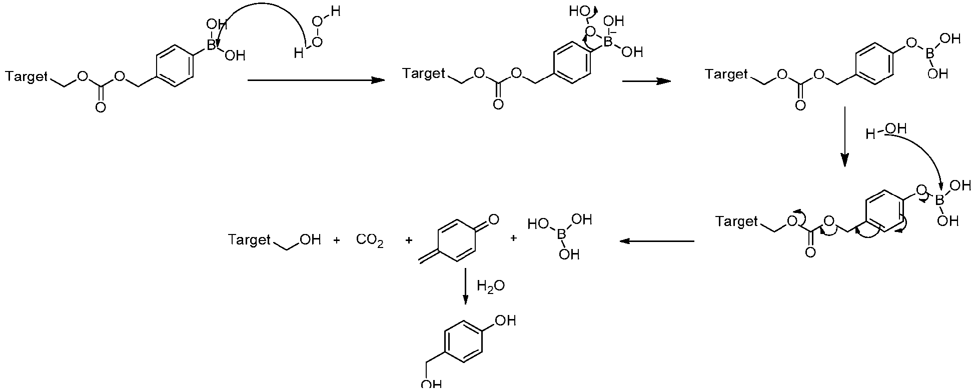

### EXAMPLE 2

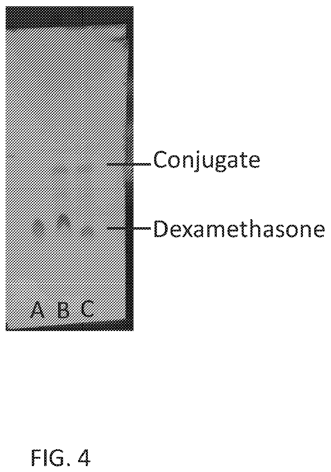

Compound A was synthesized by conjugating dexamethasone with a [4-(2-carboxymethyl)phenyl]boronic acid. The stability of the conjugate was tested in phosphate buffered saline (PBS)/H₂O, and no release of the dexamethasone was observed. When compound A was subjected to a known amount of hydrogen peroxide, release of dexamethasone was observed as confirmed by thin layer chromatography (TLC) analysis as shown in FIG. 4. Illustrated below is the reaction scheme showing dexamethasone sequestered when it is conjugated with a boronic acid moiety in form of Compound A. Upon addition of hydrogen peroxide, the boronic acid reacts with the hydrogen peroxide and releases the dexamethasone.

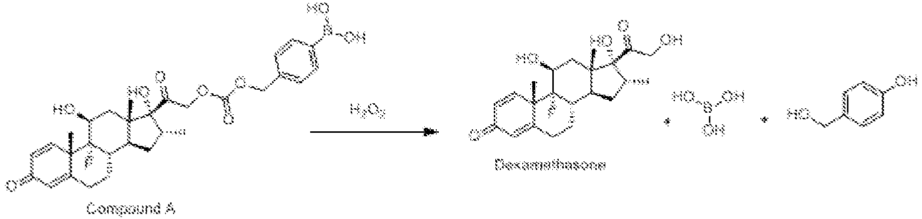

### EXAMPLE 3

A sensor including a sensor housing, a hydrogel on at least a portion of the sensor housing, indicator molecules contained in the hydrogel, and Pt sputtered on at least a portion of the hydrogel has a useful life of about 90 days if implanted in a human patient. The sensor is further protected by a boronic acid-drug conjugate covering at least a portion of the surface of the hydrogel and the further protected sensor has a useful life of at least 180 days when implanted in a human patient.

Embodiments of the present invention have been fully described above with reference to the drawing figures. Although the invention has been described based upon these preferred embodiments, it would be apparent to those of skill in the art that certain modifications, variations, and alternative constructions could be made to the described embodiments within the spirit and scope of the invention. For example, although in some embodiments, the analyte sensor 100 may be an optical sensor, this is not required, and, in one or more alternative embodiments, the analyte sensor may be a different type of analyte sensor, such as, for example, an electrochemical sensor, a diffusion sensor, or a pressure sensor. Also, although in some embodiments, the analyte sensor 100 may be an implantable sensor, this is not required, and, in some alternative embodiments, the analyte sensor may be a transcutaneous sensor having a wired connection to an external transceiver. For example, in some alternative embodiments, the analyte sensor 100 may be located in or on a transcutaneous needle *(e.g.,* at the tip thereof). In these embodiments, instead of wirelessly communication using an antenna (e.g., inductive element 114), the analyte sensor may communicate with the external transceiver using one or more wires connected between the external transceiver and a transceiver transcutaneous needle including the analyte sensor. For another example, in some alternative embodiments, the analyte sensor may be located in a catheter *(e.g.,* for intravenous blood glucose monitoring) and may communicate (wirelessly or using wires) with an external transceiver.

## Claims

1. A sensor (100) for measurement of an analyte in a medium within a living animal, the sensor comprising:
a sensor housing (102);
an analyte indicator (106) covering at least a portion of the sensor housing, wherein the analyte indicator includes molecules configured to reversibly bind the analyte; and
a boronic acid-drug conjugate comprising a drug configured to reduce deterioration of the analyte indicator caused by degradative species, conjugated to a boronic acid moiety incorporated in and/or in close proximity to the analyte indicator, wherein the boronic acid-drug conjugate is configured to release the drug in the presence of the degradative species.

2. The sensor of claim 1, further comprising at least one drug eluting polymer matrix covering at least a portion of the sensor housing, wherein the boronic acid-drug conjugate is dispersed within the drug eluting polymer matrix;
wherein the sensor preferably further comprises at least one of the following features:
the drug eluting polymer matrix has a preformed shape, and the preformed shape preferably is a ring, a sleeve, a conformal shell, a cylinder, or a monolith; and
the drug eluting polymer matrix is adjacent to the analyte indicator.

3. The sensor of claim 1 or 2, wherein the sensor includes at least one of the following features:
the boronic acid-drug conjugate is configured to reduce oxidation of the analyte indicator; and
the boronic acid-drug conjugate is configured to interact or react with a degradative species without compromising signal integrity or performance of the sensor device, and the degradative species is hydrogen peroxide, a reactive oxygen species, a reactive nitrogen species, or a free radical.

4. The sensor of any one of claims 1-3, wherein the drug of the boronic acid-drug conjugate is conjugated to the boronic acid moiety via covalent bonds that are stable in the absence of a degradative species and/or that break in the presence of a degradative species and release the drug.

5. The sensor of claim 1 or 2, wherein the one or more boronic acid-drug conjugates are co-monomers with the analyte indicator, and the analyte indicator is preferably in a hydrogel.

6. The sensor of any one of claims 1-5, wherein the drug is an anti-inflammatory drug, the anti-inflammatory drug is preferably a non-steroidal anti-inflammatory drug, and the non-steroidal anti-inflammatory drug is preferably acetylsalicylic acid or isobutylphenylpropanoic acid.

7. The sensor of any one of claims 1-5, wherein the drug is a glucocorticoid, dexamethasone, triamcinolone, betamethasone, methylprednisolone, beclometasone, fludrocortisone, a derivative thereof, an analog thereof, or a combination of two or more thereof.

8. The sensor of any one of claims 1-7, wherein the analyte indicator is a graft including the indicator molecules.

9. The sensor of any one of claims 1-8, further comprising a layer of a catalyst capable of converting hydrogen peroxide into water and oxygen on at least a portion of the analyte indicator.

10. The sensor of any one of claims 1-9, further comprising a membrane covering at least a portion of the analyte indicator, wherein the membrane is preferably a porous, opaque diffusion membrane.

11. The sensor of any one of claims 1-10, wherein the boronic acid-drug conjugate is formed by conjugating a boronic acid compound of Formula I to the drug, and Formula I is: 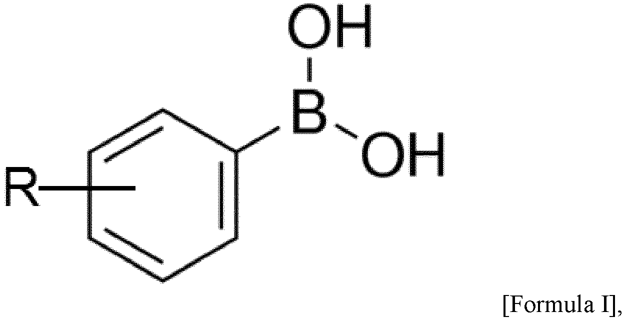 wherein one or more R substituent may be independently selected from hydrogen, hydroxyl, an alkyl group, an alkenyl group, an alkynyl group, a halo group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂, wherein R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

12. The sensor of any one of claims 1-11, wherein the boronic acid-drug conjugate is: 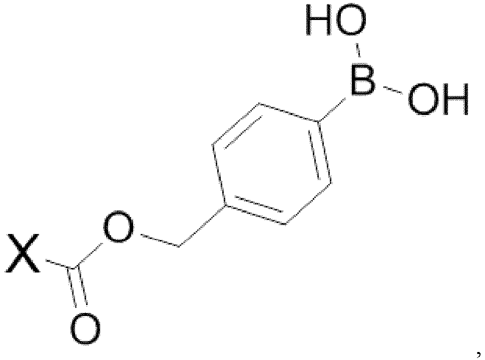 wherein X is the drug or a linking moiety connecting the boronic acid moiety to the drug, wherein the linking moiety is a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂, wherein R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

13. The sensor of any one of claims 1-11, wherein the drug of the boronic acid-drug conjugate is conjugated with one or more of the following compounds either directly or via a linking moiety: 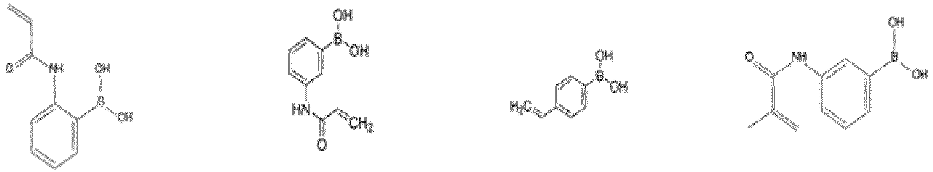 wherein, in a conjugate having the linking moiety, the linking moiety is a hydroxyl, an alkyl group, an alkenyl group, an alkynyl group, an aldehyde group, a carboxylate group, an alkoxy group, a carboxyl group, an ester, an amide group, an imide group, a carbonyl group, an amino group, an aryl group, a heteroaryl, a cyclic group, and/or NR₁R₂, wherein R and R₂ may be identical or different and each may represent a hydrogen atom, a hydroxyl group, an alkyl group, an alkoxy group, an amino group, an aryl group, a heteroaryl, a cyclic group, a carboxylic acid, a vinyl group, an acrylate group, an acryloyl group, or a methacrylate group.

14. The sensor of any one of claims 1-11, wherein the drug of the boronic acid-drug conjugate is conjugated to [4-(2-carboxymethyl)phenyl]boronic acid.

15. A method of fabricating the sensor of any one of claims 1-14, the method comprising:
applying the analyte indicator to the sensor housing of the sensor such that the applied analyte indicator covers at least the portion of the sensor housing, wherein the analyte indicator includes the indicator molecules that reversibly bind the analyte, and wherein:
(i) one or more boronic acid-drug conjugates configured to release a drug and reduce deterioration of the analyte indicator caused by degradative species are incorporated in the analyte indicator;
(ii) a drug eluting polymer matrix of the sensor comprises one or more boronic acid-drug conjugates configured to release a drug and reduce deterioration of the analyte indicator caused by the degradative species;
or
(iii) both (i) and (ii).

## Patentansprüche

1. Sensor (100) zur Messung eines Analyten in einem Medium innerhalb eines lebenden Tieres, wobei der Sensor umfasst:
ein Sensorgehäuse (102);
einen Analytindikator (106), der mindestens einen Abschnitt des Sensorgehäuses bedeckt, wobei der Analytindikator Moleküle umfasst, die dazu eingerichtet sind, den Analyten reversibel zu binden; und
ein Boronsäure-Wirkstoff-Konjugat, umfassend einen Wirkstoff, der dazu eingerichtet ist, durch abbauende Spezies verursachte Verschlechterung des Analytindikators zu reduzieren, konjugiert mit einer Boronsäuregruppe, eingebaut in den und/oder in großer Nähe zu dem Analytindikator, wobei das Boronsäure-Wirkstoff-Konjugat dazu eingerichtet ist, den Wirkstoff in der Gegenwart der abbauenden Spezies freizusetzen.

2. Sensor nach Anspruch 1, ferner umfassend mindestens eine wirkstoffeluierende Polymermatrix, die mindestens einen Abschnitt des Sensorgehäuses bedeckt, wobei das Boronsäure-Wirkstoff-Konjugat in der wirkstoffeluierenden Polymermatrix dispergiert ist;
wobei der Sensor vorzugsweise mindestens eines der folgenden Merkmale umfasst:
die wirkstoffeluierende Polymermatrix hat eine vorgebildete Form, und die vorgebildete Form ist vorzugsweise ein Ring, eine Hülse, eine konforme Hülle, ein Zylinder oder ein Monolith; und
die wirkstoffeluierende Polymermatrix ist angrenzend an den Analytindikator.

3. Sensor nach Anspruch 1 oder 2, wobei der Sensor mindestens eines der folgenden Merkmale umfasst:
das Boronsäure-Wirkstoff-Konjugat ist dazu eingerichtet, Oxidation des Analytindikators zu reduzieren; und
das Boronsäure-Wirkstoff-Konjugat ist dazu eingerichtet, mit einer abbauenden Spezies zu wechselwirken oder zu reagieren, ohne eine Signalintegrität oder Leistung der Sensorvorrichtung zu beeinträchtigen, und die abbauende Spezies ist Wasserstoffperoxid, eine reaktive Sauerstoffspezies, eine reaktive Stickstoffspezies oder ein freies Radikal.

4. Sensor nach einem der Ansprüche 1-3, wobei der Wirkstoff des Boronsäure-Wirkstoff-Konjugats an die Boronsäuregruppe über kovalente Bindungen konjugiert ist, die in der Abwesenheit einer abbauenden Spezies stabil sind und/oder in der Gegenwart einer abbauenden Spezies brechen und den Wirkstoff freisetzen.

5. Sensor nach Anspruch 1 oder 2, wobei das eine oder die mehreren Boronsäure-Wirkstoff-Konjugate Co-Monomere mit dem Analytindikator sind und der Analytindikator vorzugsweise in einem Hydrogel vorliegt.

6. Sensor nach einem der Ansprüche 1-5, wobei der Wirkstoff ein entzündungshemmender Wirkstoff ist, der entzündungshemmende Wirkstoff vorzugsweise ein nichtsteroidaler entzündungshemmender Wirkstoff ist und der nichtsteroidale entzündungshemmende Wirkstoff vorzugsweise Acetylsalicylsäure oder Isobutylphenylpropionsäure ist.

7. Sensor nach einem der Ansprüche 1-5, wobei der Wirkstoff ein Glukokortikoid, Dexamethason, Triamcinolon, Betamethason, Methylprednisolon, Beclometason, Fludrocortison, ein Derivat davon, ein Analogon davon oder eine Kombination aus zwei oder mehr davon ist.

8. Sensor nach einem der Ansprüche 1-7, wobei der Analytindikator ein Transplantat ist, das die Indikator-Moleküle umfasst.

9. Sensor nach einem der Ansprüche 1-8, ferner umfassend eine Schicht eines Katalysators, der in der Lage ist, auf mindestens einem Abschnitt des Analytindikators Wasserstoffperoxid in Wasser und Sauerstoff umzuwandeln.

10. Sensor nach einem der Ansprüche 1-9, ferner umfassend eine Membran, die mindestens einen Abschnitt des Analytindikators bedeckt, wobei die Membran vorzugsweise eine poröse, undurchsichtige Diffusionsmembran ist.

11. Sensor nach einem der Ansprüche 1-10, wobei das Boronsäure-Wirkstoff-Konjugat durch Konjugieren einer Boronsäureverbindung der Formel I mit dem Wirkstoff gebildet wird und Formel I 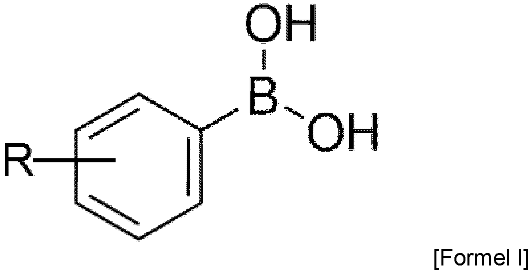 ist,
wobei ein oder mehrere R-Substituenten unabhängig ausgewählt sein können aus Wasserstoff, Hydroxyl, einer Alkylgruppe, einer Alkenylgruppe, einer Alkinylgruppe, einer Halogengruppe, einer Aldehydgruppe, einer Carboxylatgruppe, einer Alkoxygruppe, einer Carboxylgruppe, einem Ester, einer Amidgruppe, einer Imidgruppe, einer Carbonylgruppe, einer Aminogruppe, einer Arylgruppe, einem Heteroaryl, einer cyclischen Gruppe und/oder NR₁R₂, wobei R₁ und R₂ identisch oder unterschiedlich sein können und jeweils ein Wasserstoffatom, eine Hydroxylgruppe, eine Alkylgruppe, eine Alkoxygruppe, eine Aminogruppe, eine Arylgruppe, ein Heteroaryl, eine cyclische Gruppe, eine Carbonsäure, eine Vinylgruppe, eine Acrylatgruppe, eine Acryloylgruppe oder eine Methacrylatgruppe darstellen können.

12. Sensor nach einem der Ansprüche 1-11, wobei das Boronsäure-Wirkstoff-Konjugat 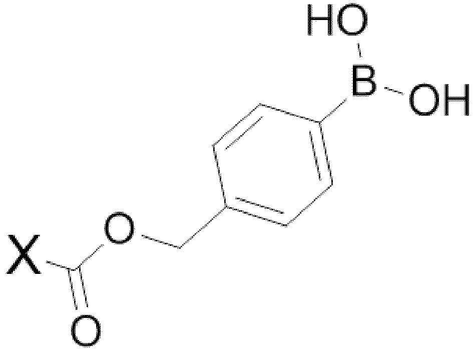 ist,
wobei X der Wirkstoff oder eine Linkergruppe ist, die die Boronsäuregruppe mit dem Wirkstoff verbindet, wobei die Linkergruppe eine Hydroxylgruppe, eine Alkylgruppe, eine Alkenylgruppe, eine Alkinylgruppe, eine Aldehydgruppe, eine Carboxylatgruppe, eine Alkoxygruppe, eine Carboxylgruppe, ein Ester, eine Amidgruppe, eine Imidgruppe, eine Carbonylgruppe, eine Aminogruppe, eine Arylgruppe, ein Heteroaryl, eine cyclische Gruppe und/oder NR₁R₂ ist, wobei R₁ und R₂ identisch oder unterschiedlich sein können und jeweils ein Wasserstoffatom, eine Hydroxylgruppe, eine Alkylgruppe, eine Alkoxygruppe, eine Aminogruppe, eine Arylgruppe, ein Heteroaryl, eine cyclische Gruppe, eine Carbonsäure, eine Vinylgruppe, eine Acrylatgruppe, eine Acryloylgruppe oder eine Methacrylatgruppe darstellen können.

13. Sensor nach einem der Ansprüche 1-11, wobei der Wirkstoff des Boronsäure-Wirkstoff-Konjugats mit einer oder mehreren der folgenden Verbindungen entweder direkt oder über eine Linkergruppe konjugiert ist: 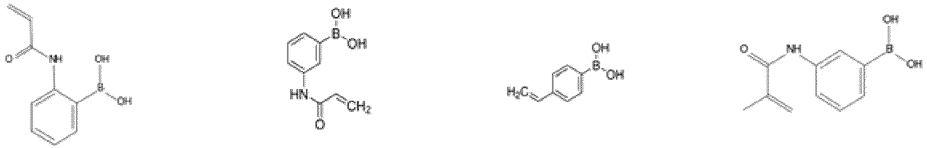 wobei in einem Konjugat mit der Linkergruppe die Linkergruppe eine Hydroxylgruppe, eine Alkylgruppe, eine Alkenylgruppe, eine Alkinylgruppe, eine Aldehydgruppe, eine Carboxylatgruppe, eine Alkoxygruppe, eine Carboxylgruppe, ein Ester, eine Amidgruppe, eine Imidgruppe, eine Carbonylgruppe, eine Aminogruppe, eine Arylgruppe, ein Heteroaryl, eine cyclische Gruppe und/oder NR₁R₂ ist, wobei R₁ und R₂ identisch oder unterschiedlich sein können und jeweils ein Wasserstoffatom, eine Hydroxylgruppe, eine Alkylgruppe, eine Alkoxygruppe, eine Aminogruppe, eine Arylgruppe, ein Heteroaryl, eine cyclische Gruppe, eine Carbonsäure, eine Vinylgruppe, eine Acrylatgruppe, eine Acryloylgruppe oder eine Methacrylatgruppe darstellen können.

14. Sensor nach einem der Ansprüche 1-11, wobei der Wirkstoff des Boronsäure-Wirkstoff-Konjugats an [4-(2-Carboxymethyl)phenyl]boronsäure konjugiert ist.

15. Verfahren zur Herstellung des Sensors nach einem der Ansprüche 1-14, wobei das Verfahren umfasst:
Aufbringen des Analytindikators auf das Sensorgehäuse des Sensors, so dass der aufgebrachte Analytindikator mindestens den Abschnitt des Sensorgehäuses bedeckt, wobei der Analytindikator die Indikator-Moleküle umfasst, die den Analyten reversibel binden, und wobei:
(i) ein oder mehrere Boronsäure-Wirkstoff-Konjugate, die dazu eingerichtet sind, einen Wirkstoff freizusetzen und durch abbauende Spezies verursachte Verschlechterung des Analytindikators zu reduzieren, in den Analytindikator eingebaut sind;
(ii) eine wirkstoffeluierende Polymermatrix des Sensors ein oder mehrere Boronsäure-Wirkstoff-Konjugate umfasst, die dazu eingerichtet sind, einen Wirkstoff freizusetzen und durch abbauende Spezies verursachte Verschlechterung des Analytindikators zu reduzieren; oder
(iii) sowohl (i) als auch (ii).

## Revendications

1. Capteur (100) pour la mesure d'un analyte dans un milieu à l'intérieur d'un animal vivant, le capteur comprenant :
un boîtier de capteur (102) ;
un indicateur d'analyte (106) recouvrant au moins une partie du boîtier de capteur, dans lequel l'indicateur d'analyte comprend des molécules conçues pour se lier de manière réversible à l'analyte ; et
un conjugué acide boronique-médicament comprenant un médicament conçu pour réduire la détérioration de l'indicateur d'analyte causée par des espèces de dégradation, conjugué à une fraction acide boronique incorporée dans et/ou à proximité immédiate de l'indicateur d'analyte, dans lequel le conjugué acide boronique-médicament est conçu pour libérer le médicament en présence des espèces de dégradation.

2. Capteur selon la revendication 1, comprenant en outre au moins une matrice polymère d'élution de médicament recouvrant au moins une partie du boîtier de capteur, dans lequel le conjugué acide boronique-médicament est dispersé au sein de la matrice polymère d'élution de médicament ;
dans lequel le capteur comprend de préférence, en outre, au moins l'une des caractéristiques suivantes :
la matrice polymère d'élution de médicament présente une forme préformée, et la forme préformée est de préférence un anneau, un manchon, une coque conforme, un cylindre ou un monolithe ; et
la matrice polymère d'élution de médicament est adjacente à l'indicateur d'analyte.

3. Capteur selon la revendication 1 ou 2, dans lequel le capteur inclut au moins l'une des caractéristiques suivantes :
le conjugué acide boronique-médicament est conçu pour réduire l'oxydation de l'indicateur d'analyte ; et
le conjugué acide boronique-médicament est conçu pour interagir ou réagir avec une espèce de dégradation sans compromettre l'intégrité du signal ou les performances du dispositif capteur, et l'espèce de dégradation est le peroxyde d'hydrogène, une espèce réactive de l'oxygène, une espèce réactive de l'azote ou un radical libre.

4. Capteur selon l'une quelconque des revendications 1 à 3, dans lequel le médicament du conjugué acide boronique-médicament est conjugué à la fraction acide boronique par des liaisons covalentes qui sont stables en l'absence d'une espèce de dégradation et/ou qui se rompent en présence d'une espèce de dégradation et libèrent le médicament.

5. Capteur selon la revendication 1 ou 2, dans lequel le ou les conjugués acide boronique-médicament sont des comonomères avec l'indicateur d'analyte, et l'indicateur d'analyte est de préférence dans un hydrogel.

6. Capteur selon l'une quelconque des revendications 1 à 5, dans lequel le médicament est un médicament anti-inflammatoire, le médicament anti-inflammatoire est de préférence un médicament anti-inflammatoire non stéroïdien, et le médicament anti-inflammatoire non stéroïdien est de préférence l'acide acétylsalicylique ou l'acide isobutylphénylpropanoïque.

7. Capteur selon l'une quelconque des revendications 1 à 5, dans lequel le médicament est un glucocorticoïde, la dexaméthasone, la triamcinolone, la bétaméthasone, la méthylprednisolone, la béclométasone, la fludrocortisone, un dérivé de ceux-ci, un analogue de ceux-ci ou une combinaison de deux ou plus de ceux-ci.

8. Capteur selon l'une quelconque des revendications 1 à 7, dans lequel l'indicateur d'analyte est une greffe incluant les molécules d'indicateur.

9. Capteur selon l'une quelconque des revendications 1 à 8, comprenant en outre une couche d'un catalyseur capable de convertir le peroxyde d'hydrogène en eau et en oxygène sur au moins une partie de l'indicateur d'analyte.

10. Capteur selon l'une quelconque des revendications 1 à 9, comprenant en outre une membrane recouvrant au moins une partie de l'indicateur d'analyte, dans lequel la membrane est de préférence une membrane de diffusion poreuse et opaque.

11. Capteur selon l'une quelconque des revendications 1 à 10, dans lequel le conjugué acide boronique-médicament est formé par conjugaison d'un composé d'acide boronique de formule I au médicament, et la formule I est : 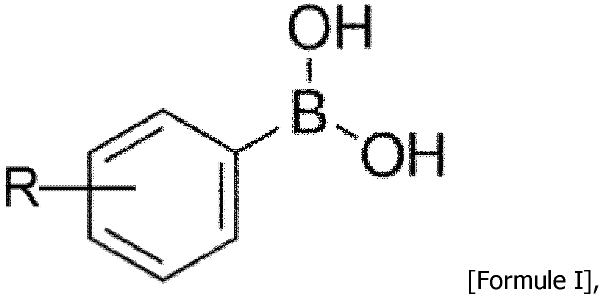 dans laquelle un ou plusieurs substituants R peuvent être indépendamment choisis parmi l'hydrogène, l'hydroxyle, un groupe alkyle, un groupe alcényle, un groupe alcynyle, un groupe halogéno, un groupe aldéhyde, un groupe carboxylate, un groupe alcoxy, un groupe carboxyle, un ester, un groupe amide, un groupe imide, un groupe carbonyle, un groupe amino, un groupe aryle, un hétéroaryle, un groupe cyclique et/ou NR₁R₂, dans lequel R et R₂ peuvent être identiques ou différents et peuvent chacun représenter un atome d'hydrogène, un groupe hydroxyle, un groupe alkyle, un groupe alcoxy, un groupe amino, un groupe aryle, un hétéroaryle, un groupe cyclique, un acide carboxylique, un groupe vinyle, un groupe acrylate, un groupe acryloyle ou un groupe méthacrylate.

12. Capteur selon l'une quelconque des revendications 1 à 11, dans lequel le conjugué acide boronique-médicament est : 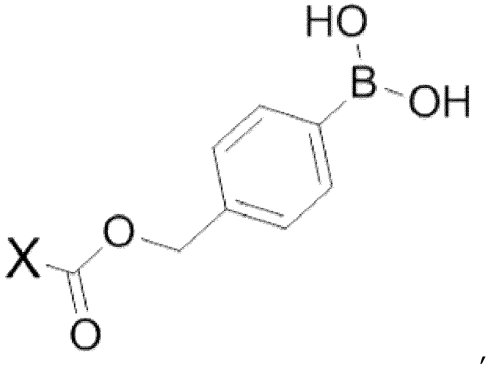
dans laquelle X représente le médicament ou une fraction de liaison reliant la fraction acide boronique au médicament,
dans laquelle la fraction de liaison est un hydroxyle, un groupe alkyle, un groupe alcényle, un groupe alcynyle, un groupe aldéhyde, un groupe carboxylate, un groupe alcoxy, un groupe carboxyle, un ester, un groupe amide, un groupe imide, un groupe carbonyle, un groupe amino, un groupe aryle, un hétéroaryle, un groupe cyclique et/ou NR₁R₂, dans lequel R et R₂ peuvent être identiques ou différents et peuvent chacun représenter un atome d'hydrogène, un groupe hydroxyle, un groupe alkyle, un groupe alcoxy, un groupe amino, un groupe aryle, un hétéroaryle, un groupe cyclique, un acide carboxylique, un groupe vinyle, un groupe acrylate, un groupe acryloyle ou un groupe méthacrylate.

13. Capteur selon l'une quelconque des revendications 1 à 11, dans lequel le médicament du conjugué acide boronique-médicament est conjugué à un ou plusieurs des composés suivants, directement ou par l'intermédiaire d'une fraction de liaison : 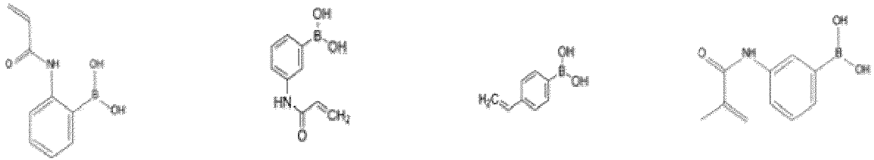 dans lesquels, dans un conjugué comportant la fraction de liaison, la fraction de liaison est un hydroxyle, un groupe alkyle, un groupe alcényle, un groupe alcynyle, un groupe aldéhyde, un groupe carboxylate, un groupe alcoxy, un groupe carboxyle, un ester, un groupe amide, un groupe imide, un groupe carbonyle, un groupe amino, un groupe aryle, un hétéroaryle, un groupe cyclique et/ou NR₁R₂, dans lequel R et R₂ peuvent être identiques ou différents et peuvent chacun représenter un atome d'hydrogène, un groupe hydroxyle, un groupe alkyle, un groupe alcoxy, un groupe amino, un groupe aryle, un hétéroaryle, un groupe cyclique, un acide carboxylique, un groupe vinyle, un groupe acrylate, un groupe acryloyle ou un groupe méthacrylate.

14. Capteur selon l'une quelconque des revendications 1 à 11, dans lequel le médicament du conjugué acide boronique-médicament est conjugué à l'acide [4-(2-carboxyméthyl)phényl]boronique.

15. Procédé de fabrication du capteur selon l'une quelconque des revendications 1 à 14, le procédé comprenant :
l'application de l'indicateur d'analyte sur le boîtier de capteur du capteur de manière à ce que l'indicateur d'analyte appliqué recouvre au moins la partie du boîtier de capteur, dans lequel l'indicateur d'analyte inclut les molécules d'indicateur qui se lient de manière réversible à l'analyte, et dans lequel :
(i) un ou plusieurs conjugués acide boronique-médicament conçus pour libérer un médicament et réduire la détérioration de l'indicateur d'analyte causée par des espèces de dégradation sont incorporés dans l'indicateur d'analyte ;
(ii) une matrice polymère d'élution de médicament du capteur comprend un ou plusieurs conjugués acide boronique-médicament conçus pour libérer un médicament et réduire la détérioration de l'indicateur d'analyte causée par les espèces de dégradation ; ou
(iii) à la fois (i) et (ii).
